# EUROPÄISCHE PATENTANMELDUNG

(11) **EP 1 201 227 A2**
(43) Veröffentlichungstag der Anmeldung: **02.05.2002**
(21) Anmeldenummer: 01123102.4
(22) Anmeldetag: 12.12.1995
(51) Int. Cl.: A61K 7/48

(54) **Verwendung von Flavonoiden als immunmodulierende oder immunschützende Agenzien in kosmetischen und dermatologischen Zubereitungen**

(30) Priorität: 13.12.1994 DE 4444238
(62) Teilanmeldung aus: 95942130.6
(71) Anmelder: Beiersdorf Aktiengesellschaft, 20245 Hamburg (DE)
(72) Erfinder: Untiedt, Sven, 20259 Hamburg (DE); Stäb, Franz, 21379 Echem (DE); Lanzendörfer, Ghita, 22087 Hamburg (DE)

(57) **Zusammenfassung**

Verwendung kosmetischer und dermatologischer Zubereitungen mit
einem Gehalt an einer Wirkstoffkombination, enthaltend eine Verbindung oder mehrere Verbindungen ausgewählt aus der Gruppe der Flavonoide in Kombination mit einer Verbindung oder mehreren Verbindungen ausgewählt aus der Gruppe der Zimtsäurederivate und
gegebenenfalls einem zusätzlichen Gehalt an einer Verbindung oder mehreren Verbindungen aus der Gruppe der Antioxidantien zur Behandldung oder prophylaktischen Behandlung der durch UVB-Strahlung induzierten Immunsuppression, insbesondere zur Behandlung oder prophylaktischen Behandlung entzündlicher, allergischer oder autoimmunreaktiver Erscheinungen und zum Schutz von Zellen, welche an der Immunantwort der Haut beteiligt sind.

## Beschreibung

Verwendung von Flavonoiden als immunmodulierende oder immunschützende Agenzien in kosmetischen und dermatologischen Zubereitungen

Die vorliegende Erfindung betrifft Wirkstoffe und Zubereitungen, solche Wirkstoffe enthaltend, zur kosmetischen oder dermatologischen Behandlung und/oder Prophylaxe entzündlicher, allergischer oder autoimmunreaktiver Erscheinungen, sowie zum Schutz von Zellen, welche an der Immunantwort der Haut beteiligt sind.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismen spielt.

Unter den physikalischen Umwelteinflüssen kommt dem Licht eine bedeutende Stellung zu. Die schädigende Wirkung des ultravioletten Teils der Sonnenstrahlung auf die Haut ist allgemein bekannt. Während Strahlen mit Wellenlängen kleiner als 290 nm (der sogenannte UVC-Bereich), von der Ozonschicht in der Erdatmosphäre absorbiert werden, verursachen Strahlen im Bereich zwischen 290 und 320 nm, dem sogenannten UVB-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Ultraviolettes Licht aus dem Wellenlängenbereich zwischen ca. 320 und 400 nm (UVA-Bereich) kann ebenfalls Folgeschäden der Haut hervorrufen. So ist erwiesen, daß auch UVA-Strahlung zu einer Schädigung der elastischen und kollagenen Fasern des Bindegewebes führt, was die Haut vorzeitig altern läßt (sogenanntes Photoaging), und daß sie als Ursache zahlreicher phototoxischer und photoallergischer Reaktionen zu sehen ist. Der schädigende Einfluß der UVB-Strahlung kann durch UVA-Strahlung verstärkt werden.

Bei der Entwicklung topischer Sonnenschutzmittel ist die UVB-Strahlung von besonderem Interesse, da hier das Aktionsspektrum für die akut entzündlichen Prozesse (Sonnenbrand) und chronischen Schäden (Photoaging) angesiedelt ist.

Neben diesen Effekten kann es bei UVB-Einwirkung ferner zu einer gravierenden Änderung der intraepidermalen immunologischen Situation kommen, die man als UVB-induzierte Immunsuppression bezeichnet. Dabei sind unter Umständen, je nach Strahlendosis, tiefgreifende Veränderungen der immunologischen Abläufe der Haut, mit sowohl lokalen als auch systemischen Auswirkungen, mögliche Folgen.
Immunsuppression im allgemeinen ist die Unterdrückung oder Abschwächung der Reaktivität des Immunsystems. Die UVB-induzierte Immunsuppression kann in lokale und systemische Effekte aufgegliedert werden. Letztlich umfaßt sie eine Vielzahl verschiedenster Aspekte, welche alle eine Reduktion der normalen immunologischen Abwehrmechanismen der Haut beinhalten. So wurde am Modell UVB-bestrahlter Mäuse schon sehr früh der Zusammenhang des verstärkten Tumorwachstums mit immunsuppressiver Wirkung des UVB-Lichtes in Zusammenhang gesetzt. Diese UVB-induzierte Immunsuppression wird heutzutage als Mechanismus diskutiert, mittels dessen an sich hoch immunogene, UVB-induzierte neoplastische Zellen sich der immunologischen Abwehr, und damit ihrer eigenen Zerstörung, entziehen.

Weiterhin kommt es bei UVB-Bestrahlung zu einer starken Abnahme der Kontakt-Hypersensitivitätsreaktion gegenüber manchen die Haut sensibilisierenden Agenzien. Der Grund dafür könnte in einer drastischen Verminderung der Anzahl der epidermalen Langerhanszellen liegen und/oder einer Änderung deren Morphologie und Funktionalität. Langerhanszellen aber stellen den afferenten Arm der immunologischen Abwehr der Haut dar. Ferner unterbleiben effektive Abwehrreaktionen gegen infektiöse Keime wie z.B. Candida albicans oder Herpes simples Virus.

Schließlich wird als Folge einer dermatologisch relevanten UVB-Exposition die Expression des "Intercellular Adhesion Molecule-1" auf epidermalen Keratinocyten supprimiert. Dieses zelloberflächenständige Glycoprotein (auch ICAM-1 genannt) ist eine der wichtigsten zellulären Kommunikationsstrukturen, über die direkte Zell-Zell-Kontakte zwischen epidermalen Keratinocyten und Leukocyten wie z.B. T-Lymphocyten und Monocyten reguliert werden.

Die UVB-induzierte Immunsuppression betrifft also ein breites Spektrum immunologischer Dysfunktionen, welche in einer Reduktion der normalerweise ablaufenden Abwehrreaktionen resultieren.

Es ist zwar üblich, gegen die unmittelbare Einwirkung der ultravioletten Strahlung absorbierende Agentien, nämlich die üblichen Lichtschutzsubstanzen zu verwenden.

Zum Schutz gegen die Strahlen des UVA-Bereichs werden beispielsweise vorwiegend Derivate des Dibenzoylmethans verwendet.

Zum Schutz gegen UVB-Strahlung sind viele Verbindungen bekannt, bei denen es sich vorwiegend um Derivate des 3-Benzylidencamphers, der 4-Aminobenzoesäure, der Zimtsäure, der Salicylsäure, des Benzophenons sowie auch des 2-Phenylbenzimidazols handelt.

Es war auch weiterhin bekannt, Radikalfänger als gegen durch UV-Strahlung induzierte photoxidative Erscheinungen in der Haut wirkende Agenzien einzusetzen. Bekanntlich handelt es sich bei solchen photochemischen Reaktionsprodukten vorwiegend um radikalische Verbindungen, beispielsweise Hydroxylradikale oder Superoxidradikalanionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere reaktive Sauerstoff-Spezies. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

So ist bereits vorgeschlagen worden, Vitamin E bzw. Vitamin E-Ester, Substanzen mit bekannter antioxidativer Wirkung, in Lichtschutzformulierungen einzusetzen. Der Hintergrund war allerdings stets UV-Schutz durch Lichtabsorption oder Schutz gegen photooxidative Prozesse. Außerdem war die Wirksamkeit von Vitamin E aus topischen Vehikeln schwach. Auch eine hohe Dosierung brachte keine Abhilfe, da besonders bei Vitamin E eine eher prooxidative Wirkung erzielt wurde.

Auch Kombinationen von 2,4-O-Furfurylidensorbitol, Thiolen und Vitamin E zur Stärkung des hauteigenen Immunsystems, welche in PCT/DE93/00773 angeführt werden, sind hinter den Erwartungen zurückgeblieben.

Aufgabe der vorliegenden Erfindung war es daher und diese Aufgabe wird erfindungsgemäß gelöst, Wirkstoffe und Zubereitungen, solche Wirkstoffe enthaltend, zur Verfügung zu stellen, mit Hilfe derer
- eine wirksamere Prophylaxe gegen die UVB-lmmunsuppression bewirkt werden kann.
- das durch die UVB-lmmunsuppression bereits geschädigte Immunsystem wieder gekräftigt werden kann.

Die erfindungsgemäßen Wirkstoffe und Zubereitungen wirken in dieser Weise.

Es war überraschend und für den Fachmann nicht vorauszusehen, daß die kosmetischen oder dermatologischen Zubereitungen zur Behandlung und/oder Prophylaxe der durch UVB-Strahlung induzierten Immunsuppression, gekennzeichnet durch einen therapeutisch oder kosmetisch wirksamen Gehalt an nachfolgend spezifizierten Substanzen
sowie die
Verwendung kosmetisch oder dermatologisch unbedenklicher, nachfolgend spezifizierter Substanzen zur kosmetischen oder dermatologischen Behandlung und/oder Prophylaxe der durch UVB-Strahlung induzierten Immunsuppression die Lösung dieser Aufgaben darstellen würde.

Insbesondere vorteilhaft werden die erfindungsgemäßen Substanzen gewählt aus der Gruppe der Flavonoide und ihrer Glucoside, aus der Gruppe der Zimtsäurederivate sowie aus der Gruppe der Tocopherole und ihrer Derivate.

Die Schrift JP-OS Hei-06-138,941 beschreibt zwar orale Zubereitungen mit einem Gehalt an wasserlöslichen Glucosiden, welche beispielsweise gewählt werden können aus der Gruppe α-Glucosylrutin, α-Glucosylmyrictrin, α-Glucosylisoquercitrin und α-Glucosylquercitrin. Die Schrift JP-OS Hei-04-363,395 beschreibt ein Verfahren, die Zersetzung von Parfümbestandteilen zu verhindern, welche sich unter anderem durch einen Zusatz an α-Glucosylrutin zu den entsprechenden Zubereitungen auszeichnet. Ferner beschreiben die Schriften EP-OS 586 303 und EP-OS 595 694 die Verwendung von Flavonoïden als Antioxidantien bzw. Lichtschutzsubstanzen in Kosmetika. Weiterhin ist aus US-PS 4,144,325 und 4,248,861 sowie aus zahlreichen anderen Dokumenten bekannt, Vitamin E in kosmetischen und dermatologischen Lichtschutzformulierungen einzusetzen. Die erfindungsgemäße Verwendung des Vitamins E und seiner Derivate zur kosmetischen oder dermatologischen Prophylaxe der durch UVB-Strahlung induzierten Immunsuppression wurde indes nicht durch den Stand der Technik nahegelegt.

Kein Hinweis ist diesen Schriften aber zu entnehmen, welcher in die Richtung der vorliegenden Erfindung weisen könnte.

Die vorstehenden Aufgaben werden gemäß der Erfindung gelöst.

Gegenstand der Erfindung ist die Verwendung kosmetischer und dermatologischer Zubereitungen mit
a) einem Gehalt an einer Verbindung oder mehreren Verbindungen aus der Gruppe der Flavonoide oder mit
b) einem Gehalt an einer Wirkstoffkombination, enthaltend eine Verbindung oder mehrere Verbindungen ausgewählt aus der Gruppe der Flavonoide in Kombination mit einer Verbindung oder mehreren Verbindungen ausgewählt aus der Gruppe der Zimtsäurederivate und
c) gegebenenfalls einem zusätzlichen Gehalt an einer Verbindung oder mehreren Verbindungen aus der Gruppe der Antioxidantien zur Behandldung oder prophylaktischen Behandlung der durch UVB-Strahlung induzierten Immunsuppression, insbesondere zur Behandlung oder prophylaktischen Behandlung entzündlicher, allergischer oder autoimmunreaktiver Erscheinungen und zum Schutz von Zellen, welche an der Immunantwort der Haut beteiligt sind.

Wirkstoffkombinationen b), deren Verwendung und Zubereitungen, die diese enthalten werden bevorzugt.

Gegenstand der Erfindung ist auch die Verwendung der erfindungsgemäßen Wirkstoffe für die genannten Zwecke und ihre Verwendung als immunmodulierende oder immunschützende Wirkstoffe, insbesondere in kosmetischen und dermatologischen Zubereitungen.

Die erfindungsgemäßen Wirkstoffe und Zubereitungen dienen zum Schutz immunkompetenter Zellen wie Langerhanszellen und zum Schutz von Zellbestandteilen.

Topische Zubereitungen werden bevorzugt.

Bevorzugte erfindungsgemäße Flavonoide sind beispielsweise hydroxylierte Flavone, Flavanone, Isoflavone oder Chalcone und jeweils auch deren Glycoside, aber auch diese nicht hydroxylierten Grundstrukturen bzw. Stammsubstanzen.

Die erfindungsgemäßen Flavonoide werden im folgenden auch mit A), die erfindungsgemäßen Zimtsäurederivate mit B) und die erfindungsgemäßen Antioxidantien auch mit C) bezeichnet.

Erfindungsgemäß werden die Flavonoide A) bevorzugt gewählt aus der Gruppe der Substanzen der generischen Strukturformeln 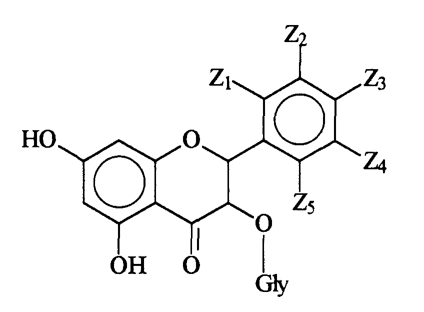 und 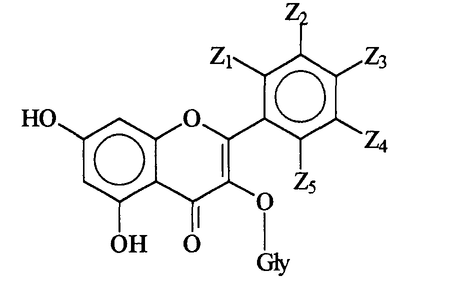 wobei Z₁ - Z₅ unabhängig voneinander gewählt werden aus der Gruppe H, OH und O-Alkyl, wobei die Alkylgruppen verzweigt und unverzweigt sein und 1- 18 C-Atome aufweisen können, und wobei Gly gewählt wird aus der Gruppe der Mono-und Oligoglycosidreste oder auch H darstellen kann. Bevorzugte Glycosidreste sind die für Gly₁ - Gly₃ nachstehend angegebenen.

Weitere erfindungsgemäßge Flavonoide werden vorteilhaft gewählt aus der Gruppe der Substanzen der folgenden Formeln: 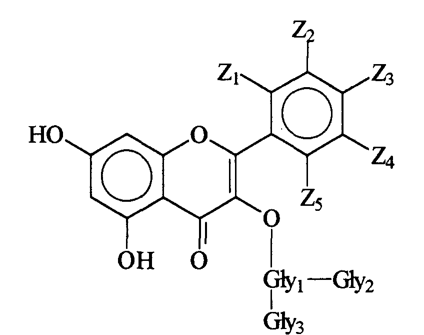 sowie 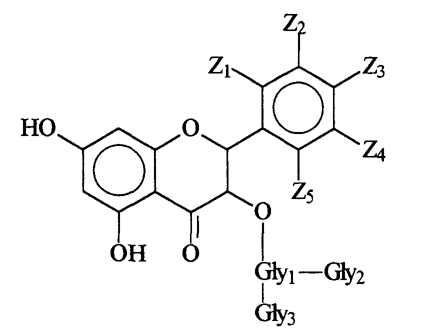 worin Z₁ - Z₅ die oben angegebenene Bedeutung haben und Gly₁, Gly₂ und Gly₃ Monoglycosidreste darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Apiosyl, Arabinosyl, Biosidyl, Galactosyl, Gulosyl, Glucoronidyl, Idosyl, Mannosyl, Talosyl und Xylosyl sind gegebenenfalls vorteilhaft zu verwenden. Es kann auch erfindungsgemäß vorteilhaft sein, Pentosylreste zu verwenden

Besonders vorteilhaft im Sinne der vorliegenden Erfindung ist, das oder die Flavonglycoside zu wählen aus der Gruppe alpha-Glucosylrutin, alpha-Glucosylmyrictrin, alpha-Glucosylisoquercitrin und alpha-Glucosylquercitrin.

Darüberhinaus sind besonders erfindungsgemäß bevorzugt Verbindungen wie alpha-Glycosylrutin, alpha-Glycosylhesperidin, alpha-Glycosylnaringin, alpha-Mannosylrutin, alpha-Rhamnosylrutin.

Es kann ebenfalls vorteilhaft sein auf die oben erwähnten glycosidischen Reste Gly₁₋₃ zu verzichten und die nicht substituierten Flavonoide (Gly₁₋₃ = H), wie z. B. Quercitin, zu verwenden. Ebenso kann es von Vorteil sein Flavonoide zu verwenden, deren Glucosid-Rest über phenolische OH-Funktionen an C7, C4', C3' oder C5' gebunden ist.

Weiterhin kann es von Vorteil sein, Flavonoide zu verwenden, deren phenolische OH-Funktion an C9 frei vorliegt (sogenannte Chalkone). Insbesondere ist es vorteilhaft aus dieser Gruppe Neohesperidin-Dihydrochalkon zu verwenden.

Vorteilhaft im Sinne der vorliegenden Erfindung ist es, das oder die Flavonoide zu wählen aus der Gruppe Quercitin, Rutin, Chrysin, Kaempferol, Myricetin, Rhamnetin, Apigenin, Luteolin, Naringin, Hesperidin, Naringenin, Hesperitin, Morin, Phloridzin, Diosmin, Fisetin, Vitexin, Neohesperidin Dihydrochalkon, Flavon, Glucosylrutin und Genistein.

Die erfindungsgemäß besonders bevorzugten Flavonoide sind Chrysin, Naringin, Hesperidin, Naringenin, Hesperetin, Morin, Phloridzin, Diosmin, Neohesperidin-Dihydrochalkon, Flavon und insbesondere alpha-Glucosylrutin der Formel

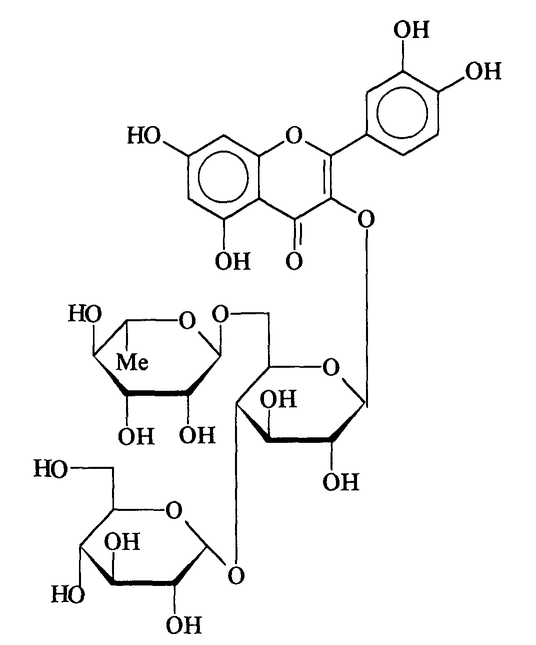

Darüberhinaus kann es vorteilhaft sein im Sinne der Erfindung handelsübliche, flavonoidhaltige Pflanzenextrakte zu verwenden. Bei diesen kann es sich um nach den üblichen Methoden gewonnene wassrig-alkoholische bzw. wässrig-glykolische Extrakte sowie trockene Extrakte handeln.

Insbesondere als vorteilhaft haben sich erwiesen: Zitrusfruchtschalen- oder-kernextrakt (z. B. Citricidal/Fa. Synthapharm), Soyaextrakt (z. B. Phytodermin/Fa. Chem. Laboratorium Dr. Kurt Richter GmbH), Sophora Japonica-Extrakt (z. B. Sophorine/Fa. Solabia), Frauendistelextrakt (z. B. Psoralen Silymarin/Fa. Mani GmbH Chemische Produkte), Katzenpfötchenblütenextrakt, Spinatextrakt und ein gemischter Pflanzenextrakt aus Passionsblumen, schwarzen Johannisbeeren und Weinbättern (AE Complex/Fa. Solabia).

Geeignete Zimtsäurederivate sind z.B. Hydroxyzimtsäuren und deren Derivate, wobei die Derivate z.B. die im folgenden definierten sein können.

Erfindungsgemäß können Zimtsäurederivate der allgemeinen Formel 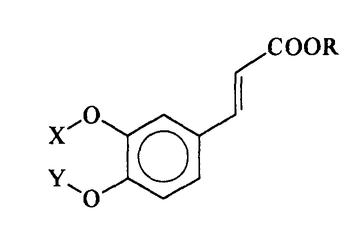 und/oder wirksame Mengen an Zimtsäurederivaten der allgemeinen Formel 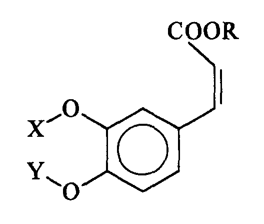 verwendet werden, wobei die Gruppen X, Y und R unabhängig voneinander gewählt werden können aus der Gruppe H, verzweigtes bzw. unverzweigtes Alkyl mit 1 - 18 C-Atomen.

Es können die Säuren oder deren Salze verwendet werden, vorzugsweise die physiologisch verträgliche Salze, beispielsweise wasserlösliche Salze (Natrium-, Kaliumsalze).

Als besonders vorteilhaftes Zimtsäurederivat im Sinne der vorliegenden Erfindung wird die Ferulasäure angesehen. Ferulasäure (4-Hydroxy-3-methoxyzimtsäure, Kaffeesäure-3-methylether) ist durch die Strukturformel 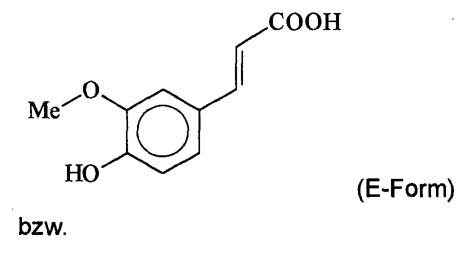 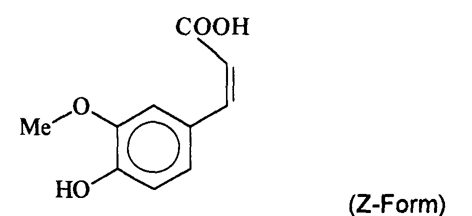 gekennzeichnet. Sie ist in Pflanzen weit verbreitet und kommt z.B. in Rüben, Getreide und dem Milchsaft der namensgebenden Doldenblütlern *Ferula asafoetida* und *Ferula nartex* vor. Die E-Form ist unter Normalbedingungen ein farblos-kristalliner Festkörper, die Z-Form liegt unter Normalbedingungen als gelbliches Öl vor.

Im Sinne der vorliegenden Erfindung ist bevorzugt E-Ferulasäure zu verwenden. Es ist jedoch gegebenenfalls auch von Vorteil, Z-Ferulasäure bzw. beliebige Gemische aus E- und Z-Ferulasäure einzusetzen.

Eine weiteres erfindungsgemäß bevorzugtes Derivat der Zimtsäure ist die Kaffeesäure, welche sich durch die Struktur 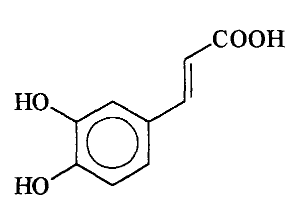 auszeichnet. Sie ist eine weit verbreitete Pflanzensäure und z.B. in Kaffee, Tabak, Mohn und Löwenzahn enthalten.

Es ist auch gegebenenfalls vorteilhaft Pflanzenextrakte mit einem Gehalt an erfindungsgemäßen Zimtsäurederivaten, insbesondere Ferulasäure und/oder Kaffeesäure, zu verwenden.

Unter dem Begriff "Derivate der Kaffeesäure oder Ferulsäure" sind zu verstehen ihre kosmetisch oder pharmakologisch unbedenklichen Ester, Salze und Basenaddukte, insbesondere solche, wie sie bei den Zimtsäurederivaten vorstehend beschrieben sind.

Bevorzugte erfindungsgemäße Kombinationen sind Kombinationen von einem oder mehreren Stoffen aus der Gruppe der oben aufgeführten Flavonoide oder Kombinationen von einem oder mehreren Vertretern der Flavonoide mit einem Derivat der Zimtsäure oder auch die Kombination mit mehreren Zimtsäurederivaten.

Erfindungsgemäß besonders bevorzugft sind die Kombinationen von Flavonoiden, Flavonglucosiden bzw. flavonoidhaltigen pflanzlichen Extrakten mit Ferulasäure sowie die Kombination von synthetisch modifizierten, insbesondere glykosylierten Flavonoiden wie alpha-Glukosylrutin mit Zimtsäurederivaten.

Das Gewichtsverhältnis der Zimtsäurederivate zu dem oder den Flavonoiden beträgt vorteilhaft 25 : 1 bis 1 : 25, bevorzugt 5 : 1 bis 1 : 5, insbesondere bevorzugt etwa 2 : 1 bis 1 : 2.

Besonders bevorzugt werden Zubereitungen mit Kombinationen b), die alpha-Glucosylrutin und/oder Ferulasäure enthalten.

In den erfindungsgemäßen Zubereitungen können als alleinige Wirkstoffe die Verbindungen der Gruppe A oder die der Kombination der Wirkstoffe A) und b) vorliegen.

Die erfindungsgemäßen Zubereitungen können aber bevorzugt neben den Wirkstoffen A) oder der Kombination von A) und B) auch noch einen Gehalt an einem Antioxidans oder mehreren Antioxidantien C) haben.

Vorteilhaft können die erfindungsgemäßen Antioxidantien C) aus der Gruppe der Tocopherole und deren Derivaten ausgewählt werden. Die Tocopherole, auch Vitamin E genannt, leiten sich vom Grundkörper Tocol (2-Methyl-2-(4,8,12-trimethyltridecyl)chroman-6-ol) ab. Dem natürlich am häufigsten vorkommenden und bedeutendsten α-Tocopherol kommt die Konfiguration 2R,4'R,8'R zu. Es wird gelegentlich auch RRR-α-Tocopherol genannt.

Die erfindungsgemäß bevorzugten Tocopherolderivate sind das α-Tocopherol und seine Ester, insbesondere das α-Tocopherylacetat. Ester von Säuren mit 2 - 18, insbesondere 2 - 8 C-Atomen werden bevorzugt.

Sofern Vitamin E und/oder dessen Derivate das oder die Antioxidantien darstellen, ist vorteilhaft, deren jeweilige Konzentrationen aus dem Bereich von 0,001- 10 Gew.-%, bezogen auf das Gesamtgewicht der Formulierung, zu wählen.

Weiterhin ist es vorteilhaft Antioxidantien C) aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil, Thioredoxin, und Glutathion, ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Vitamin A und Derivate (Vitamin-A-palmitat) sowie Koniferylbenzoat des Benzoeharzes, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe zu verwenden.

Die Menge der vorgenannten Antioxidantien C) (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die erfindungsgemäßen kosmetischen und/oder dermatologischen Formulierungen können wie üblich zusammengesetzt sein und zur Prophylaxe und/oder zur Behandlung der Haut im Sinne einer dermatologischen Behandlung oder einer Prophylaxe und/oder Behandlung im Sinne der Kosmetik dienen. Sie können aber auch in Schminkprodukten in der dekorativen Kosmetik eingesetzt werden.

Die erfindungsgemäßen kosmetischen und dermatologischen Zubereitungen enthalten bevorzugt 0,001 Gew.-% bis 30 Gew.-%, vorzugsweise 0,01 Gew.-% bis 10 Gew.-%, insbesondere aber 0,1 Gew.-% bis 6 Gew.-%, bezogen auf das Gesamtgewicht eines oder mehrerer erfindungsgemäßer Stoffe A) oder der Kombination von A) und B).

Es ist erfindungsgemäß vorteilhaft, Kombinationen aus mehreren Antioxidantien zu verwenden, insbesondere, wenn wenigstens eine der Komponenten gewählt wird aus der Gruppe der Flavonoide bzw. deren Glucoside und der Zimtsäurederivate ausgewählt wird.

Vorteilhaft ist es insbesondere, Kombinationen aus mindestens einer Verbindung aus den Flavonoiden A) bzw. deren Derivaten, mindestens einer Verbindung aus den Zimtsäurederivaten B) sowie Vitamin E bzw. seinen Derivaten C) zu verwenden.

Vorteilhaft ist es insbesondere, Kombinationen aus synthetisch modifizierten, z. B. glykosylierten Flavonoiden bzw. deren Derivaten, Ferulasäure sowie Vitamin E bzw. seinen Derivate zu verwenden. Ebenso ist es insbesondere vorteilhaft, Kombinationen aus natürlichen Flavonoiden bzw. deren Derivaten, Zimtsäure und deren Derivaten sowie Vitamin E bzw. seinen Derivate zu verwenden.

Die Gewichtsanteile von Wirkstoffen der Gruppe der Flavonoide bzw. deren Derivaten und der Gruppe der Zimtsäure bzw. ihrer Derivate können in Kombinationen in einem weiten Verhältnisbereich variiert werden. Vorzugsweise beträgt das Gewichts-Verhältnis der Wirkstoffe 20:1 bis 1:20, insbesondere 10:1 bis 1:10, besonders bevorzugt 2:1 bis 1:2.

Die Gewichtsanteile von Wirkstoffen der Gruppe der Flavonoide bzw. deren Derivaten und der Gruppe des Tocopherols bzw. seiner Derivate können in Kombinationen in ebenfalls einem weiten Verhältnisbereich variiert werden. Vorzugsweise beträgt das Gewichts-Verhältnis der Wirkstoffe 20:1 bis 1:20, insbesondere 10:1 bis 1:10, besonders bevorzugt 2:1 bis 1:2.

Bei Verwendung von Kombinationen von Wirkstoffen der Gruppe der Flavonoide bzw. deren Derivaten und der Gruppe der Zimtsäure bzw. ihrer Derivate mit der Gruppe des Tocopherols bzw. seiner Derivate können die Gewichts-Verhältnisse vorzugsweise in den folgenden Grenzen variiert werden: 20:1 bis 1:20, insbesondere 10:1 bis 1:10, besonders bevorzugt 2:1 bis 1:2.

Zur kosmetischen oder dermatologischen Behandlung und/oder Prophylaxe der durch UVB-Strahlung induzierten Immunsuppression, zum Schutz immunkompetenter Zellen wie Langerhanszellen bzw. zum Schutz von Zellbestandteilen werden die erfindungsgemäßen Zubereitungen, vorzugsweise Kombinationen aus Flavonoiden bzw. deren Derivaten, Zimtsäure und deren Derivaten sowie Vitamin E bzw. dessen Derivate in der für Kosmetika oder Dermatika üblichen Weise auf die Haut in ausreichender Menge aufgebracht.

Kosmetische Zubereitungen gemäß der Erfindung zum Schutze der Haut können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, eine wasserfreie Salbe, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. UV/A- und UV/B- Filter, Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, oder flüssige Triglceride natülicher Herkunft
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, monoethyl- oder -monobutylether, Diethylenglykolmonomethyl- oder-monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Wasserfreie kosmetische und dermatologische Zubereitungen wie Salben oder Hautöle gemäß der Erfindung sind vorteilhaft und enthalten z. B. die genannten Fette, Öle, Silikonöle, Wachse und anderen Fettkörper.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.
Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy) -1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Wirkstoffen verwendet werden können, soll selbstverständlich nicht limitierend sein.

Gegenstand der Erfindung ist auch die Kombination eines oder mehrerer erfindungsgemäßer Wirkstoffen mit einem oder mehreren UVB-Filtern bzw. erfindungsgemäße kosmetische oder dermatologische Zubereitungen, welches auch einen oder mehrere UVB-Filter enthalten.

Es kann auch von Vorteil sein, einen oder mehrere erfindungsgemäße Wirkstoffe mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn die erfindungsgemäßen Wirkstoffe mit UVA- und UVB-Filtern kombiniert werden.

Kosmetische Zubereitungen, erfindungsgemäße Wirkstoffe enthaltend, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und/oder UVB-Filter und Pigment bzw. Zubereitungen, die diese Kombination enthalten, sind Gegenstand der Erfindung. Es können die für die vorstehenden Kombinationen genannten Mengen verwendet werden.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

| Beispiel 1 | |
|---|---|
| W/O Creme | |
| | Gew.-% |
| Paraffinöl | 10,00 |
| Petrolatum | 4,00 |
| Wollwachsalkohol | 1,00 |
| PEG-7 Hydriertes Rizinusöl | 3,00 |
| Aluminumstearat | 0,40 |
| Diosmin | 0,50 |
| Ferulasäure | 0,50 |
| Glycerin | 2,00 |
| Wasser, Konservierungsmittel und Parfüm ad | ad 100,00 |

| Beispiel 2 | |
|---|---|
| W/O Lotion | |
| | Gew.-% |
| Paraffinöl | 20,00 |
| Petrolatum | 4,00 |
| Glucosesesqiisostearat | 2,00 |
| Aluminumstearat | 0,40 |
| α-Glucosylrutin | 1,00 |
| Kaffeesäure | 0,50 |
| Vitamin E Acetat | 1,00 |
| Glycerin | 5,00 |
| Wasser, Konservierungsmittel und Parfüm | ad 100,00 |

| Beispiel 3 | |
|---|---|
| O/W Lotion | |
| | Gew.-% |
| Paraffinöl | 8,00 |
| Isopropylpalmitat | 3,00 |
| Petrolatum | 4,00, |
| Cetearylalkohol | 2,00 |
| PEG-40 Rizinusöl | 0,50 |
| Natriumcetearylsulfat | 0,50 |
| Natrium Carbomer | 0,40 |
| Ferulasäure | 0,50 |
| Phloridzin | 0,20 |
| Glycerin | 3,00 |
| α-Tocopherol | 0,20 |
| Octylmethoxycinnamat | 5,00 |
| Butylmethoxydibenzoylmethan | 1,00 |
| Wasser, Konservierungsmittel und Parfüm | ad 100,00 |

| Beispiel 4 | |
|---|---|
| O/W Creme | |
| | Gew.-% |
| Paraffinöl | 7,00 |
| Avocadoöl | 4,00 |
| Glycerylmonostearat | 2,00 |
| Natriumstearat | 1,00 |
| Ferulasäure | 0,50 |
| Sophora Japonica Extrakt | 0.80 |
| (Sophorine/Fa. Solabia) | |
| Natriumphytat | 1,00 |
| Titandioxid | 1,00 |
| Natriumlactat | 3,00 |
| Glycerin | 3,00 |
| Wasser, Konservierungsmittel und Parfüm | ad 100,00 |

| Beispiel 5 | |
|---|---|
| Lippenpflegestift | |
| | Gew.-% |
| Hydriertes Rizinusöl | 4,00 |
| Ceresin | 8,00 |
| Bienenwachs | 4,00 |
| Carnaubawachs | 2,00 |
| Petrolatum | 40,00 |
| α-Glykosylrutin | 0,10 |
| β-Carotin | 0,10 |
| Kaffeesäure | 0,30 |
| Paraffinöl, Pigmente und Farbstoffe | ad 100,00 |

| Beispiel 6 | |
|---|---|
| Lippenpflegestift | |
| | Gew.-% |
| Isopropyllanolat | 10,00 |
| acetyliertes Lanolin | 4,00 |
| Bienenwachs, gebleicht | 9,00 |
| Carnaubawachs | 4,00 |
| Petrolatum | 40,00 |
| Morin | 0,10 |
| Tocopherylacetat | 0,10 |
| Ferulasäure | 0,10 |
| Paraffinöl, Pigmente und Farbstoffe | ad 100,00 |

| Beispiel 7 | |
|---|---|
| Liposomenhaltiges Gel | |
| | Gew.-% |
| Lecithin | 6,00 |
| Schibutter | 3,00 |
| Ferulasäure | 0,50 |
| Neohesperidin Dihydrochalkon | 0,10 |
| Tocopherol | 0,20 |
| Biotin | 0,08 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Wasser, Konservierungsmittel und Parfüm | ad 100,00 |

| Beispiel 8 | |
|---|---|
| Gel | |
| | Gew.-% |
| Carbopol 934 P | 2,00 |
| Triethanolamin | 3,00 |
| Ferulasäure | 0,50 |
| Hesperitin | 0,10 |
| Tocopherolacetat | 0,20 |
| Polyoxyethylensorbitanfettsäureester (Tween 20) | 0,50 |
| Glycerin | 2,00 |
| Natrium PCA | 0,50 |
| Hydrolysiertes Kollagen | 2,00 |
| Wasser, Konservierungsmittel und Parfüm | ad 100,00 |

| Beispiel 9 | |
|---|---|
| Sonnenschutzemulsion | |
| | Gew.-% |
| Cyclomethicone | 2,00 |
| Cetyldimethicone Copolyol | 0,20 |
| PEG-22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| Hesperidin | 0,50 |
| Tocopherylacetat | 0,50 |
| ZnSO₄ | 0,70 |
| Na₄EDTA | 0,30 |
| Parfum, Konservierungsmittel, Farbstoffe | nach Belieben |
| H₂O VES | ad 100,000 |

| Beispiel 10 | |
|---|---|
| Sonnenschutzemulsion | |
| | Gew.-% |
| Cyclomethicone | 2,00 |
| Cetyldimethicone Copolyol | 0,20 |
| PEG-22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| Naringin | 0,25 |
| Ferulasäure | 0,50 |
| Tocopherol | 0,50 |
| ZnSO₄ | 0,70 |
| Na₄EDTA | 0,30 |
| Parfum, Konservierungsmittel, Farbstoffe | nach Belieben |
| H₂O VES | ad 100,000 |

| Beispiel 11 | |
|---|---|
| Sonnenschutzemulsion | |
| | Gew.-% |
| Cyclomethicone | 2,00 |
| Cetearylalkohol + | |
| PEG-40-hydriertes Rizinusöl + | |
| Natrium Cetearylsulfat | 2,50 |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,50 |
| Sophora Japonica Extrakt | |
| (Sophorine/Fa. Solabia) | 0,70 |
| Tocopherylacetat | 1,00 |
| Na₃HEDTA | 1,50 |
| Parfum, Konservierungsmittel, | |
| Farbstoffe | nach Belieben |
| H₂O VES | ad 100,000 |

| Beispiel 12 | |
|---|---|
| Sonnenschutzemulsion | |
| | Gew.-% |
| Cyclomethicone | 2,00 |
| Cetearylalkohol + | |
| PEG-40-hydriertes Rizinusöl + | |
| Natrium Cetearylsulfat | 2,50 |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,75 |
| Extrakt von Passionsblumen, schwarzen Johannisbeeren und Traubenblättern (AE Complex/Fa. Solabia) | 2,50 |
| Ferulasäure | 0,30 |
| Na₃HEDTA | 1,50 |
| Parfum, Konservierungsmittel, | |
| Farbstoffe | nach Belieben |
| H₂O VES | ad 100,000 |

| Beispiel 13 | |
|---|---|
| Massagecrème | |
| | Gew.-% |
| Stearylalkohol | 2,00 |
| Petrolatum | 4,00 |
| Dimethicon | 2,00 |
| Isopropylpalmitat | 6,00 |
| Cetearylalkohol | 4,00 |
| PEG- 40 Hydriertes Rizinusöl | 2,00 |
| Tocopherol | 0,50 |
| Frauendistelextrakt | 0,30 |
| (Pronalen Silymarin/Fa. Mani GmbH) | |
| Glycerin | 3,00 |
| Wasser, Konservierungsmittel und Parfüm | ad 100,00 |

| Beispiel 14 | |
|---|---|
| Haarwasser | |
| | Gew.-% |
| Ethanol | 40,00 |
| Diisopropyladipat | 0,10 |
| Parfüm | 0,10 |
| PEG-40 hydriertes Rizinusöl | 0,20 |
| Naringenin | 0,10 |
| Tocopherylacetat | 0,10 |
| Farbstoff, Konservierungsmittel | nach Belieben |
| Wasser | ad 100 |

| Beispiel 15 | |
|---|---|
| Haarwasser | |
| | Gew.-% |
| Isopropylalkohol | 45,00 |
| Katzenpfötchenblütenextrakt (Helicrysum) | 1,00 |
| Propylenglykol | 0,50 |
| Parfüm, Farbstoff, Konservierungsmittel | nach Belieben |
| Wasser | ad 100 |

| Beispiel 16 | |
|---|---|
| Sprayformulierung | |
| | Gew.-% |
| Naringenin | 0,10 |
| Tocopherol | 0,10 |
| Ferulasäure | 0,05 |
| Ethanol | 28,20 |
| Parfüm | nach Belieben |
| Propan/Butan 25/75 | ad 100 |

### Wirkungsnachweis:

Nachfolgend sollen anhand eines Versuches die vorteilhaften Eigenschaften der vorliegenden Erfindung verdeutlicht werden.

Als Modell für die immunsupprimierende Wirkung der UVB-Strahlung wurde die UVB-Mixed-Lymphocyte-Reaction-Methode (UVB-MLR) verwendet. Die UVB-MLR ist eine Methode, um Effekte von Prüfsubstanzen auf die UVB-induzierte Suppression einer zellulären Immunantwort zu analysieren. Es handelt sich dabei um eine Modifikation der MLR, eines immunologischen in-vitro-Standardverfahrens, welches als Maß für die Aktivierung und Funktionalisierung des T-Lymphocytensystems dient.

Die Prüfsubstanzen werden hierzu den Zellkulturen in verschiedenen Konzentrationen zugefügt. Als Bestrahlungsquelle diente eine Phillips TL 20W/12-Lampe.

7,5 mJ UVB/cm², 15 mJ UVB/cm², und 30 mJ UVB/cm² wurden als Bestrahlungsdosis eingesetzt.

Mononukleäre Zellen des peripheren Blutes zweier gesunder humaner Spender werden mittels einer Dichte-Gradienten-Zentrifugation aufgereinigt und gemeinsam in Mikrotiter-Platten kultiviert. Die Einzelkultur setzt sich hierbei zusammen aus 3,0 * 10⁵ mit Mitocytin behandelten Stimulatorzellen (Spender A) und 2,5 * 10⁵ Responderzellen (Spender B) (Inkubation bei 37° C, 7,5 % CO₂, 10 % FCS (Fötales Kälberserum) in RPMI 1640 Medium).

In einer "one way"-MLR sind die Stimulatorzellen durch die Behandlung mit Mitocytin physiologisch arretiert, so daß lediglich sie als zelluläres Antigen für die Responderzellen dienen, deren Proliferation über den Einbau von ³H-Thymidin bestimmt wird. Die Responderzellen werden von den Stimulatorzellen nicht mehr als Antigen erkannt.

Der Einbau von ³H-Thymidin wird nach Separation aller Zellen, also Stimulator-und Responderzellen analysiert. Die Menge des inkorporierten ³H-Thymidins korreliert mit der Fähigkeit zur Immunantwort: je weniger ³H-Thymidin inkorporiert wird, desto stärker ist die UVB-lmmunsuppression.

Um nun den Einfluß von UV-Licht auf die Zellproliferation (Responderzellen) zu bestimmen, werden die Stimulatorzellen vor ihrer Inkubation mit den Responderzellen mit der entsprechenden UVB-Dosis bestrahlt. In entsprechenden Parallelansätzen ist die entsprechende Prüfsubstanz während der Bestrahlung im Kulturmedium zugegen.

Durch Vergleiche der in Ab- und Anwesenheit von Prüfsubstanzen erreichbaren Proliferation der Responderzellen, nach erfolgter Ko-Kultivierung mit den Stimulatorzellen, ermöglicht Aussagen über Immunprotektive Eigenschaften dieser Substanzen auf der Ebene UVB-induzierter Immunsuppression.

### Im Versuch getestete erfindungsgemäße, gegen UVB-lmmunsuppression wirksame Agentien:

Chrysin, Naringin, Hesperidin, Naringenin, Hesperitin, Morin, Phloridzin, Diosmin, Neohesperidin Dihydrochalkon, Flavon, Glucosylrutin sowie Zimtsäurederivate der allgemeinen Formel 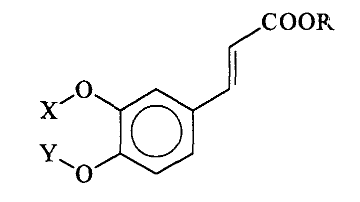 und/oder wirksame Mengen an Zimtsäurederivaten der allgemeinen Formel 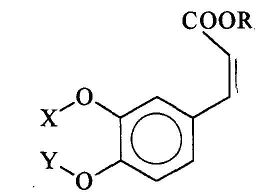 wobei die Gruppen X, Y und R unabhängig voneinander gewählt werden können aus der Gruppe H, verzweigtes bzw. unverzweigtes Alkyl mit 1- 18 C-Atomen, wurden als Prüfsubstanz verwendet.

### Ergebnis:

Es konnte bei allen vorab genannten getesteten erfindungsgemäßen, gegen UVB-lmmunsuppression wirksamen Agentien und für alle für alle drei UVB-Dosiswerte eine signifikante immunprotektive Wirkung beobachtet werden.

### Literatur zur verwendeten Methode:

Blain, B. et al. (1964), **Blood** 23, S.108,
Meo, T. et al. (1975), **Transplant. Proc.** 7, S.127
Mommaas, A. M. et al. (1990), **J. Invest. Dermatol.** 95, S.313
Marinus, C. G. et al. (1991), **J. Invest. Dermatol.** 97, S.629

## Patentansprüche

1. Verwendung kosmetischer und dermatologischer Zubereitungen mit
einem Gehalt an einer Wirkstoffkombination, enthaltend eine Verbindung oder mehrere Verbindungen ausgewählt aus der Gruppe der Flavonoide in Kombination mit einer Verbindung oder mehreren Verbindungen ausgewählt aus der Gruppe der Zimtsäurederivate und
gegebenenfalls einem zusätzlichen Gehalt an einer Verbindung oder mehreren Verbindungen aus der Gruppe der Antioxidantien zur Behandldung oder prophylaktischen Behandlung der durch UVB-Strahlung induzierten Immunsuppression, insbesondere zur Behandlung oder prophylaktischen Behandlung entzündlicher, allergischer oder autoimmunreaktiver Erscheinungen und zum Schutz von Zellen, welche an der Immunantwort der Haut beteiligt sind.

2. Verwendung gemäß Anspruch 1, **dadurch gekennzeichnet, daß** die Flavonoide gewählt sind aus der Gruppe alpha-Glucosylrutin, alpha-Glucosylmyrictrin, alpha-Glucosylisoquercitrinin und alpha-Glucosylquercitrin, Quercitin, Rutin, Chrysin, Kaempferol, Myricetin, Rhamnetin, Apigenin, Luteolin, Naringin, Hesperidin, Naringenin, Hesperitin, Morin, Phloridzin, Diosmin, Fisetin, Vitexin, Neohesperidin Dihydrochalkon, Flavon, Glucosylrutin und Genistein.

3. Verwendung gemäß Anspruch 1, **dadurch gekennzeichnet, daß** die Zubereitungen eine oder mehrere Hydroxyzimtsäuren enthalten.

4. Verwendung gemäß Anspruch 1, **dadurch gekennzeichnet, daß** Zimsäuredeivate der Formel 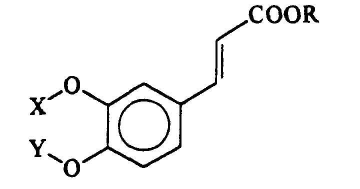 und/oder wirksame Mengen an Zimtsäurederivaten der allgemeinen Formel 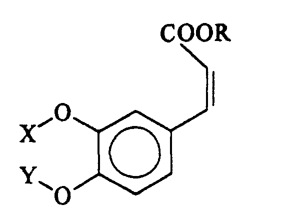 verwendet werden, wobei die Gruppen X, Y und R unabhängig voneinander gewählt werden können aus der Gruppe H, verzweigtes bzw. unverzweigtes Alkyl mit 1 - 18 C-Atomen.

5. Verwendung gemäß Anspruch 1, **dadurch gekennzeichnet, daß** Zubereitungen alpha-Glucosylrutin und Ferulasäure enthalten.

6. Kosmetische und dermatologische Zubereitungen mit
einem Gehalt an einer Wirkstoffkombination, enthaltend eine Verbindung oder mehrere Verbindungen ausgewählt aus der Gruppe der Flavonoide in Kombination mit einer Verbindung oder mehreren Verbindungen ausgewählt aus der Gruppe der Zimtsäurederivate und
gegebenenfalls einem zusätzlichen Gehalt an einer Verbindung oder mehreren Verbindungen aus der Gruppe der Antioxidantien.
